**Europäisches Patentamt**

(19) **European Patent Office**

**Office européen des brevets**

(11) Veröffentlichungsnummer: **0 166 085**
**B1**

(12) # EUROPÄISCHE PATENTSCHRIFT

(45) Veröffentlichungstag der Patentschrift:
**28.10.87**

(51) Int. Cl.⁴: **A 61 F 2/36**

(21) Anmeldenummer: **85103543.6**

(22) Anmeldetag: **15.06.82**

(60) Veröffentlichungsnummer der früheren Anmeldung
nach Art. 76 EPÜ: **0069252**

(54) **Gelenkendoprothese.**

(30) Priorität: **30.06.81 DE 3125657**
**03.05.82 DE 3216533**

(43) Veröffentlichungstag der Anmeldung:
**02.01.86 Patentblatt 86/1**

(45) Bekanntmachung des Hinweises auf die Patenterteilung:
**28.10.87 Patentblatt 87/44**

(84) Benannte Vertragsstaaten:
**AT BE CH DE FR GB IT LI NL SE**

(56) Entgegenhaltungen:
**EP - A - 0 012 146**
**DE - A - 2 839 093**
**DE - A - 2 931 750**
**FR - A - 1 083 013**
**US - A - 3 228 393**

(73) Patentinhaber: **Waldemar Link GmbH & Co,**
**Barkhausenweg 10, D-2000 Hamburg 63 (DE)**

(72) Erfinder: **Link, Helmut D., Wildstieg 14,**
**D-2000 Hamburg 65 (DE)**
Erfinder: **Keller, Arnold, An der Naherfurth 5,**
**D-2061 Kayhude (DE)**

(74) Vertreter: **Glawe, Delfs, Moll & Partner Patentanwälte,**
**Postfach 26 01 62 Liebherrstrasse 20,**
**D-8000 München 26 (DE)**

**Beschreibung**

Die Erfindung bezieht sich auf eine Gelenkendoprothese mit einem in die Höhle eines langen Knochens einzusetzenden Schaft und einer den Schaft an dessen dem Gelenk benachbarten Ende abschliessenden Auflageplatte, deren Unterfläche zur Auflage auf der formentsprechend vorbearbeiteten Resektionsfläche des Knochens bestimmt ist und Teilflächen unterschiedlicher Neigung gegenüber der Richtung des langen Knochens einschliesst, insbesondere femorale Hüftgelenkprothese.

Bei den meisten bekannten Hüftgelenkendoprothesen ist die Unterfläche der Auflageplatte eben. Da sie schräg zur Hauptbelastungsrichtung liegt, ist ihre Fähigkeit zur Kraftübertragung begrenzt. Überdies neigt sie aufgrund des Effekts der schiefen Ebene dazu, eine medial gerichtete Relativbewegung der Prothese gegenüber dem Knochen hervorzurufen. Um dies zu verhindern, hat man die Unterfläche der Auflageplatte auch schon mit Riefen oder Zähnen versehen, die beim Einsetzen der Prothese in die Knochenoberfläche eingepresst werden und damit eine Verankerung gegen seitliche Relativbewegungen ergeben sollen (DE-A 2 839 093). In der Praxis lässt sich dieses Ziel allerdings nur begrenzt erreichen, weil es sehr schwer ist, die Prothese bei der Operation so stark gegen die Resektionsfläche zu pressen, dass die Zähne hinreichend in die harte Corticalis eindringen. Es ist auch schwierig, die Resektionsfläche so genau zu formen, dass bei vorschriftmässigem Sitz der Prothese die Unterfläche der Auflageplatte genau parallel zur Resektionsfläche liegt, so dass sämtliche Zähne am Eingriff gleichmässig beteiligt werden können.

Es ist eine Hüftgelenkendoprothese bekannt (DE-A 2 931 750), bei welcher die durchgehend eben geformte Unterfläche der Auflageplatte weniger stark geneigt verlaufen soll, als dies sonst üblich ist. Jedoch erscheint die Realisierbarkeit dieses Vorschlags höchst fraglich, weil die Neigungsrichtung der Resektionsfläche nicht frei wählbar, sondern vorgegeben ist durch die Lage des grossen und kleinen Trochanter, an denen sie proximal benachbart entlanggeführt werden muss, so dass sich bei durchgehend ebener Auflageplatte im allgemeinen ein Neigungswinkel von etwa 45° ergibt.

Die Erfindung geht von der Voraussetzung aus, dass die Haupterstreckungsrichtung der Resektionsfläche durch die Lage des grossen und kleinen Trochanter vorbestimmt ist und dass ihre durchschnittliche Neigung daher nicht wesentlich veränderbar ist.

Der Erfindung liegt daher die Aufgabe zugrunde, eine Prothese der eingangs genannten Art zu schaffen, die eine sichere Kraftübertragung zwischen der Unterfläche der Auflageplatte und der Resektionsfläche des Knochens ermöglicht und bei der die mit der Unterfläche übereinstimmende Formung der Resektionsfläche erleichtert ist.

Die erfindungsgemässe Lösung besteht darin, dass die Unterfläche der Auflageplatte eine mit ringförmigen Erhöhungen und Vertiefungen versehene Rotationsfläche mit quer zu ihrer Haupterstreckungsrichtung stehender Drehachse ist.

Die übereinstimmende Gestalt der Unterfläche und der Resektionsfläche mit ringförmigen Erhöhungen und Vertiefungen ermöglicht ein formschlüssiges Ineinandergreifen und damit einen guten Prothesenhalt, insbesondere bei zementfreier Implantation. Obwohl die Gestalt der Resektionsfläche vergleichsweise kompliziert ist, lässt sie sich leicht herstellen, nämlich mittels eines chirurgischen Instruments, das aus einem rotierbaren Fräser mit einer der Unterfläche der Auflageplatte der Prothese entsprechenden Schneidenform und einem Fräserhalter besteht, der einen die Lagereinrichtung des Fräsers haltenden Schaft umfasst, der dem Schaft der Endoprothese formentsprechend gestaltet ist und vor dem Fräsvorgang in die vorgearbeitete Knochenhöhle eingesetzt werden kann, so dass der Fräser in genau derjenigen Position umläuft, in der sich bei der Prothese später die Unterfläche der Auflageplatte befinden soll. Bei einem ähnlichen, bekannten Gerät (FR-A 1 083 013), das zur Bildung einer Reaktionsebene in einem vorbestimmten Winkel zur Längsrichtung des Instruments bestimmt ist, wird ein Schaft des Instruments in eine vorher bereitete Knochenbohrung eingesetzt, die allerdings nur der Führung des Instruments dient. Der Schaft des Instruments hat daher nichts mit der später einzusetzenden Prothese zu tun. Im Unterschied dazu verwendet die Erfindung ein Instrument, dessen Schaft mit demjenigen der später einzusetzenden Prothese übereinstimmt, so dass Sicherheit für die Übereinstimmung der Position des Instruments mit der gewünschten Position der Prothese gegeben ist.

Die Übereinstimmung zwischen den Schäften der Prothese und des Fräserhalters soll möglichst gross sei, nämlich so gross, dass hinreichende Lagesicherheit für die Fräserachse besteht. Der leichteren Handhabung wegen kann jedoch der Fräserhalterschaft im allgemeinen etwas dünner als der Prothesenschaft ausgeführt sein. Auch seine Länge kann etwas geringer sein. In der Ausgestaltung der Einzelheiten kann der Fräserhalterschaft ebenfalls Abweichungen aufweisen, soweit diese die Übereinstimmung der Lage in der Markhöhle des Knochens nicht beeinträchtigt.

Das Merkmal, dass die Unterfläche der Auflageplatte eine Rotationsfläche ist, braucht nicht zu besagen, dass ihre äussere Begrenzung ebenfalls eine Rotationsfläche darstellen muss. Vielmehr kann die Unterfläche nach unterschiedlichen Seiten hin unterschiedlich weit ausladen. Beispielsweise kann vorgesehen sein, dass die Auflagefläche weiter nach medial vorragt als nach lateral. Es ist auch nicht erforderlich, dass die Rotationsachse der Auflagefläche zusammenfällt mit der Achse des Prothesenschafts oder des Prothesenhalses.

Die Erfindung wird im folgenden näher unter Bezugnahme auf die Zeichnung erläutert, die vorteilhafte Ausführungsbeispiele veranschaulicht. Darin zeigen:

Fig. 1 den proximalen Teil eines Oberschenkelknochens mit eingesetzter Prothese,

Fig. 2 einen der Fig. 1 entsprechenden Schnitt mit in den Oberschenkelknochen eingesetztem Fräsinstrument,

Fig. 3 einen Querschnitt durch den Schaft des Fräsers parallel zur Fräsebene und zum Fräser gesehen und

Fig. 4 bis 8 Querschnitte durch unterschiedliche Formen von Fräsern.

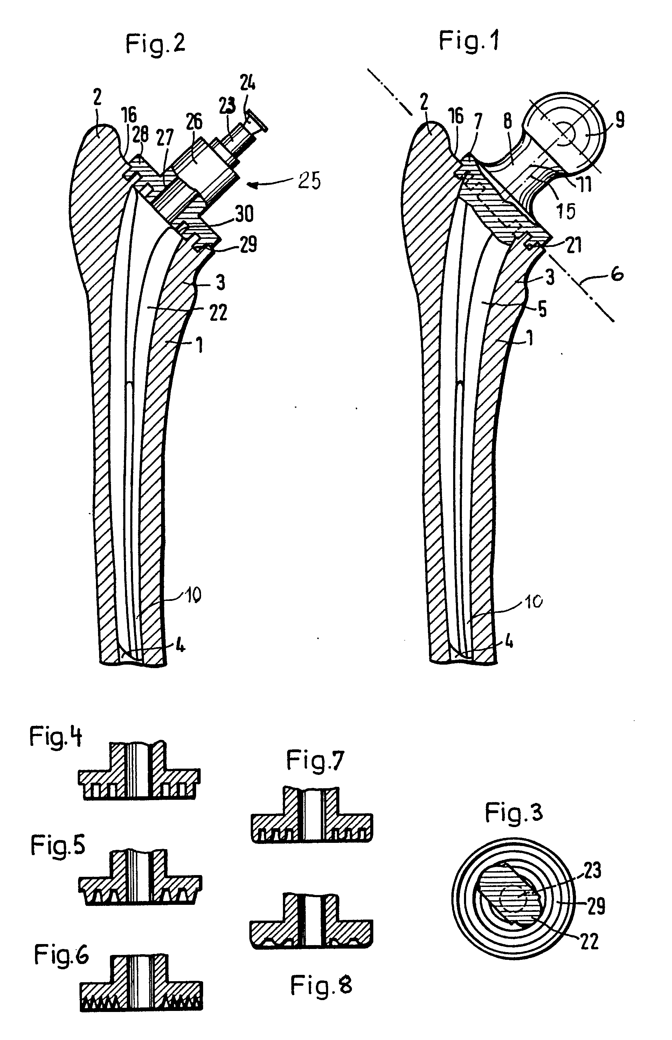

In Fig. 1 und 2 erkennt man schraffiert die Schnittfläche des Knochens 1 mit grossem Trochanter 2 und kleinem Trochanter 3 und Markhöhle 4, die zur Aufnahme des Prothesenschafts 5 vorbearbeitet ist. Der Oberschenkelhals ist längs der Ebene reseziert, deren Hauptrichtung durch die strichpunktierte Linie 6 angedeutet ist und die lateral so hoch liegt, dass der grosse Trochanter 2 erhalten bleibt, während sie medial so tief liegt, dass der Halsteil etwa die dargestellte Länge haben kann, die erforderlich ist, um dem Oberschenkelknochen hinreichende Schwenkbeweglichkeit gegenüber dem Acetabulum zu verleihen. Die Resektionslinie 6 verläuft daher etwa unter einen Winkel von 45° zur Längsrichtung des Oberschenkelknochens 1.

Die Prothese besteht aus dem Schaft 5, der Auflageplatte 7, dem Halsteil 8 und dem Gelenkkopf 9. Der Schaft ist mit oder ohne Knochenzement in die Markhöhle 4 eingesetzt und gibt in seinem distalen Abschnitt 10 die Richtung des Oberschenkelknochens an. Die Achse 11 des Halsteils 8 verläuft unter 45° zur Richtung des Oberschenkelknochens.

Die Unterfläche 21 der Auflageplatte 7 hat eine Haupterstreckungsfläche, die eben und lotrecht zur Zeichenebene ist. Sie weist eine Mehrzahl von ringförmigen Rippen und Nuten auf, deren Verlauf aus Fig. 3 deutlich wird. Die Resektionsfläche 16 des Knochens 1 enthält in dazu komplementärer Gestalt entsprechende Rippen und Nuten. Die formgenaue Übereinstimmung der Resektionsfläche und der Unterfläche der Auflageplatte erlaubt in vielen Fällen eine zementfreie Verankerung zumindest der Auflageplatte auf dem Knochen.

Die Bearbeitung der Resektionsfläche 16 erfolgt durch das in Fig. 2 dargestellte Instrument. Dieses sitzt an einem Schaft 22, der mit dem Prothesenschaft genau übereinstimmt, so dass hinreichende Sicherheit dafür besteht, dass er im Knochen dieselbe Lage einnimmt wie später der Prothesenschaft. An dem Schaft 22 ist ein Lagerzapfen 23 starr angebracht. Seine Lage stimmt überein mit der Rotationsachse der Auflagefläche 21 der Halsauflage der Prothese. Am Ende weist der Lagerzapfen 23 eine Formgebung 24 auf, die den Angriff eines Werkzeugs zum Herausziehen des Fräserhalters aus dem Knochen ermöglicht. Auf den Lagerzapfen 23 ist der Fräser 25 aufgesetzt, der aus einer Nabe 26 mit einer zum Zapfen 23 passenden Lagerbohrung 27 sowie einer Fräserplatte 28 besteht, die an ihrer Unterseite Fräszähne 29 trägt, die bei Rotation das gewünschte Flächenprofil ergeben.

Die Fig. 2 veranschaulicht den Fräser 25 in der Endstellung des Fräsvorgangs, in welcher er sich bis auf die Deckfläche 30 des Schafts 22 vorgearbeitet hat. Diese Figur veranschaulicht daher auch die Endgestalt der Resektionsfläche übereinstimmend mit Fig. 3.

Man erkennt am Vergleich der Fig. 1 und 2, dass die Achse 15 des Fräsers 25 und der Unterfläche 21 nach medial etwas verschoben ist gegenüber der Achse 11 des Prothesenhalses.

Die Fig. 4 bis 8 veranschaulichen unterschiedliche Profile, die an der Unterfläche 21 der Halsauflage im Rahmen der Erfindung verwirklichbar sind.

Die Erfindung eignet sich vornehmlich für solche Prothesen, deren Schaftform eine sichere Winkellage in der Markhöhle gewährleistet und die zu diesem Zweck nicht nur in der Lateral-Medial-Ebene, sondern auch in der Anterior-Posterior-Ebene eine charakteristische Krümmung aufweisen.

Die Beschreibung der Auflagefläche 21 der Auflageplatte der Rotationsfläche schliesst nicht aus, dass zusätzliche, zurückspringende Flächenteile vorhanden sind, die nicht als Rotationsfläche ausgebildet sind, beispielsweise radiale Nuten, die nach Füllung durch Knochengewebe oder Zement die Funktion einer Drehverhinderung gegenüber dem Knochen erfüllen.

## Patentanspruch

Gelenkendoprothese mit einem in die Höhle (4) eines langen Knochens (1) einzusetzenden Schaft (5) und einer den Schaft an dessen dem Gelenk benachbarten Ende abschliessenden Auflageplatte (7), deren Unterfläche (21) zur Auflage auf der formentsprechend vorbereiteten Resektionsfläche (6) des Knochens bestimmt ist und Teilflächen unterschiedlicher Neigung gegenüber der Dichtung des langen Knochens einschliesst, insbesondere femorale Hüftgelenkprothese, dadurch gekennzeichnet, dass die Unterfläche (21) der Auflageplatte (7) eine mit ringförmigen Erhöhungen und Vertiefungen versehene Rotationsfläche mit quer zu ihrer Haupterstreckungsrichtung (6) stehender Drehachse (15) ist.

## Claim

A joint endoprosthesis with a shaft (5) to be inserted into a cavity (4) of a long bone (1) and a bearing plate (7) terminating the shaft at its end adjoining the joint, the undersurface (21) of which bearing plate is intended to bear against the resection surface (16) of the bone, previously prepared to match the shape thereof, and includes surface portions of different inclination relative to the direction of the long bone, in particular a femoral hip-joint prosthesis, characterised in that the undersurface (21) of the bearing plate (6) is a surface of revolution provided with annular projections and recesses and having an axis of rotation (15) lying transversely to its direction of principal extension (6).

## Revendication

Endoprothèse articulaire, comprenant une tige (5) destinée à être insérée dans la cavité (4) d'un os long (1) et une plaque d'appui (7) qui termine l'extrémité de cette tige voisine de l'articulation, plaque dont la surface inférieure (21) est destinée à reposer sur la surface de résection (16) de l'os préalablement façonnée à la forme correspondante et renferme des surfaces partielles d'inclinaison différente par rapport à la direction de l'os long, en particulier prothèse fémorale de la hanche, caractérisée en ce que la surface inférieure (21) de la plaque d'appui (7) est une surface de révolution dont l'axe de révolution (15) est perpendiculaire à la direction de son étendue principale (6) et qui est munie de surélévations et de creux annulaires.

Fig. 2

Fig. 1

Fig. 4

Fig. 5

Fig. 6

Fig. 7

Fig. 8

Fig. 3